# FASCICULE DE BREVET EUROPEEN

(11) **EP 2 598 409 B1**
(45) Date de publication et mention de la délivrance du brevet: **14.03.2018**
(21) Numéro de dépôt: 11752268.0
(22) Date de dépôt: 21.07.2011
(51) Int. Cl.: B65D 47/20, B65D 81/24

(54) **EMBOUT ET RECIPIENT DE DISTRIBUTION DE LIQUIDE**
DÜSE UND CONTAINER ZUR ABGABE EINER FLÜSSIGKEIT
NOZZLE AND CONTAINER FOR DISPENSING A LIQUID

(30) Priorité: 30.07.2010 FR 1056351
(43) Date de publication de la demande: 05.06.2013
(73) Titulaire: NEMERA LA VERPILLIERE, 38290 La Verpillière (FR)
(72) Inventeur: DECOCK, Thierry, F-69002 Lyon (FR); GREVIN, Guillaume, F-38080 L'isle D'abeau (FR); JULIA, Xavier, F-38090 Villefontaine (FR); PAINCHAUD, Gaëtan, F-69340 Francheville (FR); RIMLINGER, Thierry, F-38080 L'isle D'abeau (FR)
(74) Mandataire: Vallée-Thiollier, Clémence-Olivia Laure Marie
(86) Numéro de dépôt international: PCT/FR2011/051765
(87) Numéro de publication internationale: WO 2012/013894

(56) Documents cités:
- WO-A1-97/10160
- CA-A1- 2 492 255
- US-A- 5 154 325
- US-A1- 2005 224 520

## Description

La présente invention concerne le domaine de la distribution de liquide, notamment sous forme de gouttes, dans le domaine pharmaceutique, par exemple du liquide ophtalmique, mais également dans tout autre domaine, tel que cosmétique ou alimentaire.

Plus particulièrement, l'invention concerne la distribution de liquide sans conservateur, sous forme de gouttes.

La tendance actuelle est de fournir des produits, notamment ophtalmiques, sans conservateur. Il faut donc garantir la stérilité du produit tout au long de l'utilisation du flacon contenant le liquide à délivrer.

On connait, notamment du document WO92/01625, différents dispositifs qui permettent de délivrer des gouttes de produit contenu dans un réservoir et qui évitent la contamination du liquide restant dans le flacon. Le document WO97/10160 décrit un embout de distribution de liquide selon le préambule de la revendication 1. En outre, on connait du document CA 2492255 un dispositif de distribution de liquide comprenant une pompe, dans lequel le liquide vient en contact avec une substance active présente dans la région d'un canal de sortie.

Selon un exemple, un tel dispositif de distribution de liquide comprend un réservoir et un embout de distribution monté sur le réservoir, muni d'un orifice de distribution du liquide. L'embout de distribution comprend une valve qui autorise la sortie du liquide mais empêche son entrée, ce qui limite les risques d'introduction de bactéries ou de substances contaminantes dans le flacon. Une telle valve est aussi désignée « clapet anti-retour ». L'utilisateur applique une pression sur le réservoir en le déformant et, sous l'effet de la pression, une goutte traverse la valve et se forme à la surface de l'orifice de distribution. Une fois délivrée la ou les gouttes en quantité souhaitée, la valve se referme.

Un problème avec ce type de dispositif est qu'en dépit de la fermeture de la valve, une petite quantité résiduelle de produit reste à la surface de l'embout après délivrance de la ou des gouttes dosées et que cette petite quantité résiduelle de produit peut être le siège d'une contamination qui se propage à l'intérieur du flacon.

Cette propagation est un phénomène surprenant qui contredit le principe de fonctionnement de la valve, mais que les inventeurs à la base de la présente invention ont mis en évidence.

A posteriori, les inventeurs expliquent ce phénomène par le fait que la frontière entre l'intérieur et l'extérieur du flacon n'a pas de position fixe sur la valve, mais se déplace, au gré des d'ouverture-fermeture successives, autour d'une position moyenne. De ce fait, au voisinage de cette frontière, il existe des zones limitrophes qui sont tantôt externes ou internes au flacon, c'est-à-dire en dehors de la frontière, tantôt incluses dans cette frontière.

Par ces zones limitrophes, les contaminations peuvent, après plusieurs manoeuvres d'ouverture-fermeture, trouver un chemin de migration lente, d'abord de l'extérieur du flacon vers la frontière, puis de la frontière vers l'intérieur du flacon.

La présente invention vise à résoudre cet inconvénient en proposant une valve de distribution de liquide qui garantit une absence de contamination vers l'intérieur du flacon.

A cet effet, l'invention a pour objet un embout de distribution de liquide destiné à équiper un contenant, ledit embout comprenant une valve procurant, au repos, une obturation du contenant en séparant un volume amont d'un volume aval, ladite valve étant capable de subir des manoeuvres d'ouverture par déformation élastique, ladite valve comprenant au moins deux éléments mobiles l'un par rapport à l'autre, chaque élément comportant une zone d'appui contre l'autre élément, agencée de manière que, la valve étant au repos, les zones d'appui des éléments s'appuient au moins partiellement l'une contre l'autre en formant une barrière surfacique séparant le volume amont du volume aval, l'un au moins des deux éléments étant tel qu'après une ou plusieurs manoeuvres d'ouverture, ladite barrière surfacique ne recouvre plus la même partie de la zone d'appui de l'un au moins des éléments, au moins l'un des éléments portant une matière anti-microbienne sur ou à proximité immédiate d'une partie de sa zone d'appui qui est recouverte par la barrière surfacique, caractérisé en ce que toute surface de l'embout de distribution se trouvant en contact avec le volume amont lorsque la valve est au repos est exempte de matière anti-microbienne.

Au sens de l'invention, on entend par « à proximité immédiate », le fait que la distance entre la zone d'appui et la matière anti-microbienne est inférieure à 3 mm, et de préférence inférieure à 1 mm. Il est important que cette distance soit petite pour éviter une prolifération ou un déplacement de bactéries dans le volume aval. On sait que les bactéries peuvent, lorsque les conditions sont favorables, se reproduire très rapidement. Certaines bactéries sont en outre capables de se déplacer à des vitesses surprenantes, par exemple 2 mm par minute, d'où l'importance qu'il y a à empêcher la présence de bactéries au voisinage immédiat de la valve, pour éviter leur pénétration dans le contenant.

Selon d'autres caractéristiques de l'invention, qui peuvent être prises seules ou en combinaison :
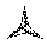 la matière anti-bactérienne est présente sous la forme d'une couche qui recouvre au moins partiellement la zone d'appui d'un des éléments ,
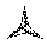 la matière anti-bactérienne est présente sous la forme d'une surépaisseur en saillie de la zone d'appui d'un des éléments la surépaisseur occupe sensiblement l'épaisseur d'un orifice de la valve,
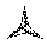 l'un des éléments a subi un traitement anti-bactérien sur une partie de son épaisseur,
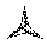 l'un des éléments est co-moulé avec une matière anti-bactérienne,
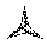 l'un des éléments porte un insert,
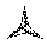 l'insert est massif en matière anti-bactérienne,
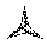 l'insert est revêtu extérieurement d'une couche de matière anti-bactérienne,
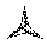 l'insert est revêtu uniquement sur sa face tournée vers l'extérieur d'une couche de matière anti-bactérienne,
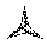 l'insert est affleurant avec la zone d'appui de l'élément mobile,
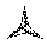 l'insert fait saillie de la zone d'appui de l'élément de manière à former un pion traversant un orifice de la valve,
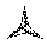 l'un des éléments est recouvert, sur une face portant de la matière anti-bactérienne, d'une couche de matière isolante non anti-bactérienne dans ses parties situées en amont de la valve,
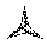 l'un des éléments est un pion d'étanchéité de forme sensiblement cylindrique et l'autre élément est une membrane comportant une partie cylindrique qui coiffe le pion et laisse autour de lui un jeu annulaire formant un canal de passage, la barrière surfacique ayant une forme sensiblement annulaire.

L'invention a également pour objet un récipient de distribution de liquide comportant un réservoir sur lequel est monté un embout de distribution tel que décrit ci-dessus, et notamment un récipient de distribution de liquide ophtalmique.

L'invention sera mieux comprise à la lecture de la description qui va suivre, ne présentant aucun caractère limitatif de la portée de l'invention mais donnée uniquement à titre d'exemples et faite en se référant aux dessins dans lesquels :
- la figure 1 est une vue en coupe d'un dispositif selon l'invention,
- les figures 2a à 2d représentent, individuellement et en perspective, différents composants du dispositif,
- la figure 3 est une vue en coupe, à plus grande échelle, de la région III de la figure 1,
- la figure 4 est une vue de dessous de la rondelle de la figure 3,
- les figures 5 à 28 sont des vues analogues à la figure 3, illustrant des variantes de réalisation de l'invention.

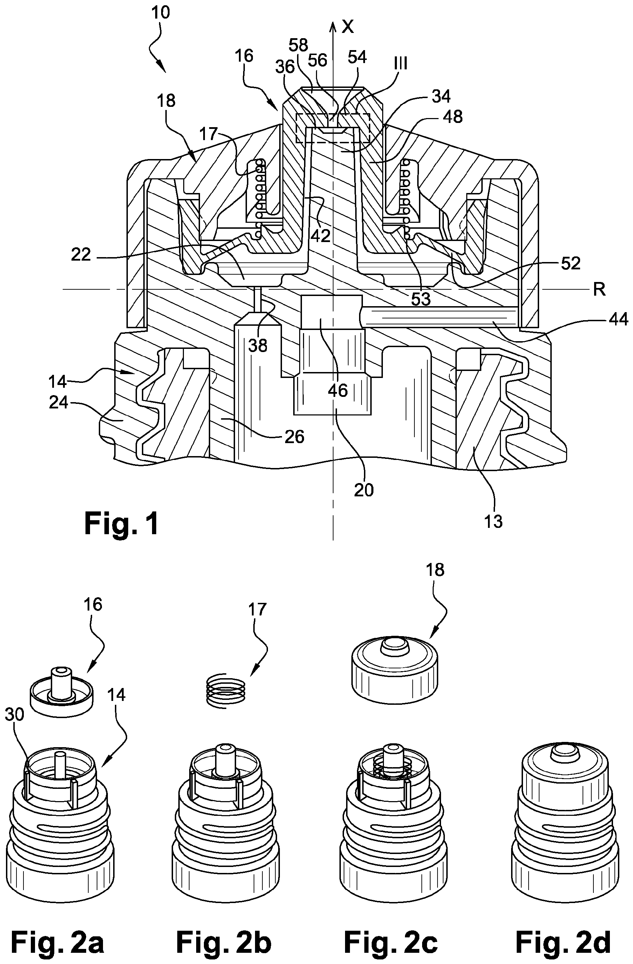

On a représenté, sur la figure 1, un embout 10 de distribution de liquide sous forme de goutte, destiné à être monté par vissage sur le col 13 d'un réservoir (dont seul le col 13 est visible sur la figure 1). Ce réservoir est un réservoir de stockage de liquide, par exemple d'un liquide pharmaceutique tel que du liquide ophtalmique. Comme cela est connu, le réservoir est déformable et peut être comprimé pour déclencher la distribution du liquide. Plus précisément, la distribution de liquide se fait par pression, de la part d'un utilisateur, sur le corps du réservoir, ce dernier pouvant présenter une certaine élasticité pour reprendre sa forme initiale après relâchement de cette pression.

L'embout de distribution 10 comprend, dans cet exemple, un support 14, une membrane 16 munie d'un orifice de distribution 58, un ressort 17, un capot 18, un canal de passage de liquide 38 du réservoir vers l'orifice de distribution 58 et un canal 44 de passage d'air dans le réservoir, canal 44 qui est obturé par un organe stérilisateur 46 perméable à l'air qui ne sera pas décrit ici.

Le support 14 comprend, dans cet exemple, une partie 24 de fixation sur le réservoir, disposée à l'extrémité proximale du support 14. Cette partie 24 comprend une jupe externe comportant un filet de vissage de façon à être vissée sur le col 13 du réservoir. La partie de fixation 24 comporte en outre une jupe interne 26 de forme tubulaire, permettant d'assurer l'étanchéité entre le réservoir et l'embout de distribution 10.

Le support 14 comporte par ailleurs un pion central d'étanchéité 34, de forme sensiblement cylindrique et s'étendant dans la direction distale, opposée à la jupe interne 24. Le pion 34 porte, sur son extrémité distale, une zone 36 d'appui du pion 34 contre la membrane 16 pour bloquer le passage de liquide en configuration de blocage. Dans cet exemple, la zone d'appui 36 prend la forme d'un bourrelet annulaire.

Le support 14 comprend également, dans cet exemple, un logement 20 formant une cavité de forme sensiblement cylindrique, cette cavité débouchant à son extrémité proximale sur le réservoir et à son extrémité distale sur le canal 38 de passage de liquide, ménagé dans le support 14 et s'étendant dans la direction longitudinale du dispositif, correspondant à la direction d'éjection. Le canal 38 débouche sur une cavité intermédiaire 22, débouchant elle-même sur un deuxième canal 42 de passage de liquide.

La membrane 16 est une pièce de révolution à géométrie particulière dont les détails non pertinents eu égard à l'invention ne seront pas décrits ici. La membrane 16 comporte une partie cylindrique 48 qui coiffe le pion 34 en laissant autour de lui un jeu annulaire formant le deuxième canal 42 précité et, à l'extrémité distale de cette partie cylindrique 48, une rondelle 54 munie d'une ouverture centrale 56 formant la petite base d'un tronc de cône ouvert vers l'extérieur, dont la grande base correspond à l'orifice de distribution 58 de la membrane. La rondelle 54 est formée d'un seul tenant avec la partie cylindrique 48. Elle repose, par sa face interne 55, sur la zone d'appui 36 du pion, grâce à l'action du ressort 17 qui repousse la partie cylindrique 48 vers la bas en exerçant une pression sur un siège 53 formé à la base de la partie cylindrique 48. Grâce à une zone flexible 52, la membrane 16 peut se déformer pour laisser sa partie cylindrique 48 coulisser axialement autour du pion 34. La face interne 55 constitue une zone d'appui de la membrane 16 contre le pion 36. Moyennant une déformation de la membrane 16, la zone d'appui 55 de la rondelle 54 peut se décoller de la zone d'appui 36 du pion et ouvrir un passage entre l'intérieur et l'extérieur du dispositif. C'est ainsi que la distribution de produit est rendue possible : une pression exercée sur les parois du flacon engendre une augmentation de la pression du liquide, lequel pousse sur la membrane 16 et la décolle du pion 34 pour arriver jusqu'à l'orifice de distribution 58. Dans les conditions normales d'utilisation du dispositif, aucune circonstance autre que celle d'une surpression dans le liquide n'est prévue pour que la rondelle 54 se décolle de la zone d'appui 36. Membrane 16 et pion 34 constituent donc deux éléments mobiles l'un par rapport à l'autre d'une valve séparant un volume amont d'un volume aval, valve qui empêche le liquide ayant quitté l'intérieur du dispositif, c'est-à-dire étant passé du volume amont au volume aval, d'y retourner.

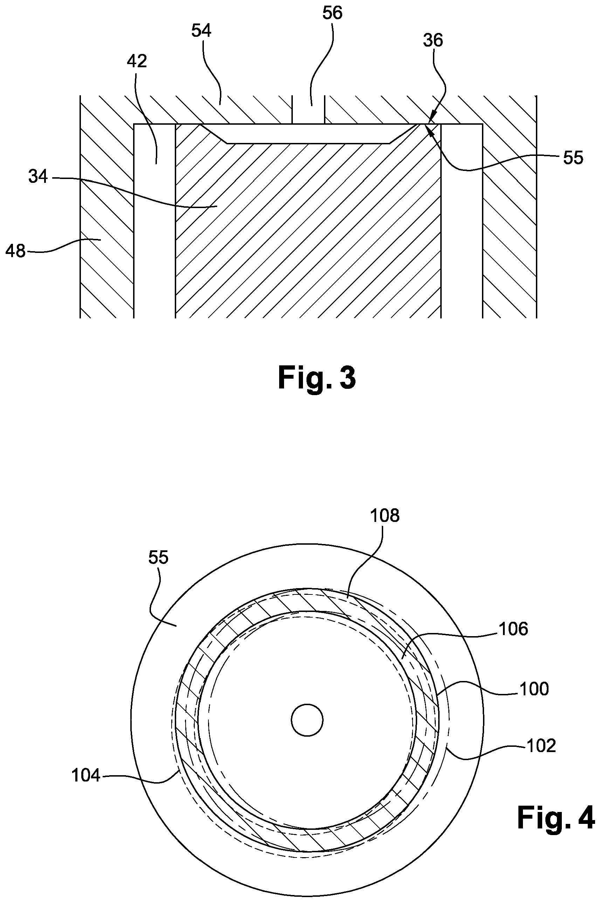

Sur les figures 3 et 4, on a représenté cette valve à plus grande échelle.

On voit que, lorsque la valve est au repos, donc fermée, la séparation entre le volume amont et le volume aval est assurée par une barrière surfacique s'étendant, côté zone d'appui 36, sur la totalité de cette zone, et, côté rondelle 54, sur la partie de la face interne 55 se trouvant au contact de la zone d'appui 36.

Mais comme le centrage de la rondelle 54 par rapport à la zone d'appui 36 n'est assuré que par l'élasticité de la membrane 16, à force d'ouvertures et fermetures successives, la fatigue de la membrane 16 ou toute autre perturbation de l'équilibre des forces au sein de la membrane peut aboutir à ce que la rondelle 54 ne revienne pas exactement dans la même position sur la zone d'appui 36. Ainsi, à une échelle micrométrique ou inférieure, mais en tout cas supérieure à l'ordre de grandeur des bactéries susceptibles de contaminer le liquide, la barrière surfacique d'obturation se déplace, au moins du côté de la face interne 55 de la rondelle 54, autour d'une position théorique moyenne et laisse, au voisinage de ses bords, des zones limitrophes qui sont tantôt internes ou externes au dispositif de distribution, c'est-à-dire exclues de la barrière surfacique, tantôt incluses dans cette barrière surfacique.

Sur la figure 4, la barrière surfacique, qui correspond à la surface selon laquelle la zone d'appui 55 et la zone d'appui 36 se touchent, est matérialisée par un anneau hachuré 100 dans une première position fermée de la valve. Après quelques ouvertures et fermetures, cet anneau prend une autre position 102, dessinée en traits mixtes. Puis, après encore quelques ouvertures et fermetures, l'anneau prend encore une autre position 104, dessinée en pointillés.

On voit bien que si une bactérie se nichait en un emplacement 106 de la zone d'appui 55 situé au voisinage immédiat de la barrière surfacique en position 102, c'est-à-dire dans le volume aval, lorsque la barrière prendrait la position 100, la bactérie ne serait plus dans le volume aval de la valve, mais dans la barrière surfacique, où elle pourrait migrer de proche en proche, à l'occasion de chaque ouverture de la valve, jusqu'à atteindre un emplacement 108 situé dans la barrière surfacique en position 100, mais dans le volume amont si la barrière surfacique adopte la position 104. La bactérie aurait alors atteint l'intérieur du réservoir et pourrait contaminer tout le liquide qu'il contient.

La zone d'appui 36 est revêtue d'une couche 110 de matériau anti-bactérien, ici à base d'ions d'argent, qui prévient toute prolifération de bactéries dans le voisinage immédiat de la barrière surfacique. Ainsi, même si une bactérie parvient à se fixer à l'emplacement 106 lorsque la barrière surfacique se trouve en position 102, elle ne pourra pas survivre si elle se retrouve, par déplacement de la barrière surfacique en position 100 ou 104, au contact de la couche anti-bactérienne de la zone d'appui 36 en position 104. La couche anti-bactérienne remplit donc efficacement sa fonction d'empêcher l'entrée dans le volume amont de contaminants pour le liquide présent dans le réservoir.

La couche anti-bactérienne n'est présente sur aucune surface de la membrane 16 ou support 14 au contact du liquide situé en amont de la valve, de manière à ce qu'aucune réaction chimique préjudiciable au liquide ne se produise dans ce volume amont.

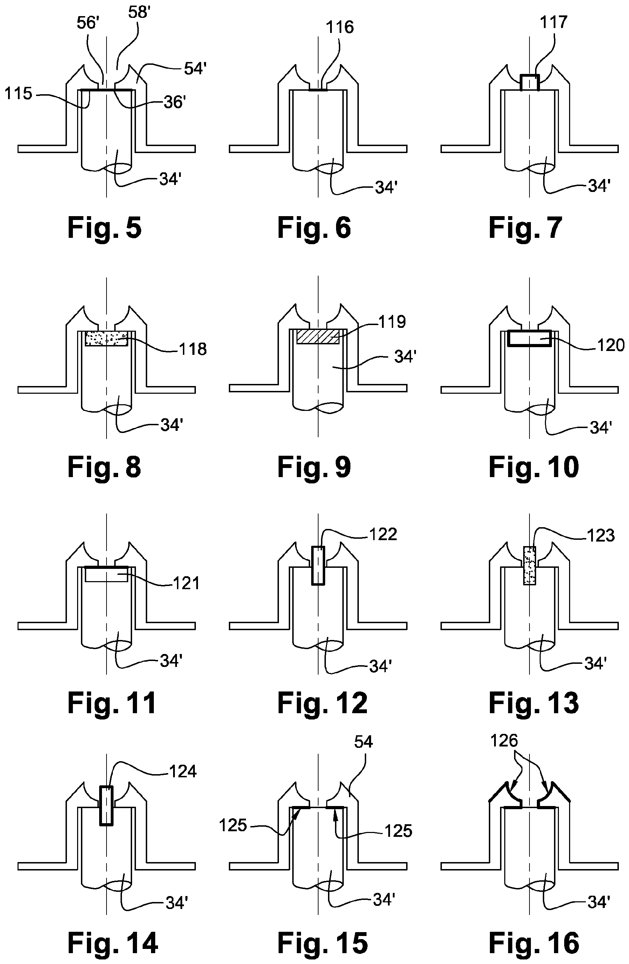

Sur les figures 5 et suivantes, on a représenté d'autres variantes de réalisation procurant les mêmes effets protecteurs. Sur ces figures, le pion 34' n'a pas de bourrelet annulaire mais présente une face supérieure plate incluant la zone d'appui 36'. La barrière surfacique est toujours un anneau, s'étendant cette fois depuis le bord périphérique du pion jusqu'au bord de l'orifice 56' de la rondelle 54'. Le tronc de cône des figures précédentes, entre les orifices 56 et 58, est remplacé par une portion de sphère joignant les orifices 56' et 58', mais définit toujours un volume mort de liquide en aval de la valve. Pour des raisons de clarté, les numéros de référence 36', 54', 56', 58' n'ont pas été reportés sur les figures 6 à 28, sur lesquelles on reconnaît facilement les éléments correspondants qui sont identiques.

Sur la figure 5, la couche anti-bactérienne 115 recouvre toute la face supérieure du pion 34'. Elle peut ainsi agir contre des bactéries qui se seraient glissées dans la barrière surfacique ainsi que contre des bactéries se développant dans le volume résiduel de liquide présent dans le volume mort aval, à proximité de l'ouverture 56'.

Sur la figure 6, la couche anti-bactérienne 116 recouvre la partie de la face supérieure du pion 34' située en regard de l'orifice 56'.

Sur la figure 7, la couche anti-bactérienne 117 constitue une surépaisseur en saillie de la face supérieure du pion 34' et occupe toute l'épaisseur de l'orifice 56'.

Sur la figure 8, le pion 34' a subi un traitement anti-bactérien sur une partie 118 de son épaisseur, dans une région centrale excluant les bords de sa face supérieure.

Sur la figure 9, le pion 34' est co-moulé avec une matière anti-bactérienne, ou porte un insert 119 massif en matière anti-bactérienne, présent dans une région centrale de sa face supérieure excluant les bords de cette face et affleurant avec ladite face.

Sur la figure 10, le pion 34' porte un insert 120 revêtu extérieurement d'une couche de matière anti-bactérienne, logé dans une région centrale de sa face supérieure excluant les bords de cette face.

Sur la figure 11, l'insert 121 est presque identique mais n'est revêtu que sur sa face tournée vers l'extérieur.

Sur la figure 12, le pion 34' porte un insert 122 massif en matière anti-bactérienne, présent dans une région centrale de sa face supérieure en regard de l'orifice 56' et faisant saillie de ladite face de manière à former un pion traversant l'orifice 56'.

Sur la figure 13, le pion 34' porte un insert 123 de forme identique au précédent mais co-moulé avec le pion 34'.

Sur la figure 14, le pion 34' porte un insert 124 presque identique à celui de la figure 12 mais qui n'est revêtu de matière anti-bactérienne que sur ses faces externes.

Sur la figure 15, la couche anti-bactérienne 125 est présente sur la rondelle 54, dans sa partie en regard du pion 34', en excluant la partie de la rondelle en regard du bord périphérique du pion 34'.

Sur la figure 16, la couche anti-bactérienne 126 s'étend, en plus de celle de la figure 15, sur le bord de la cavité sphérique et même au-delà de l'orifice 58'.

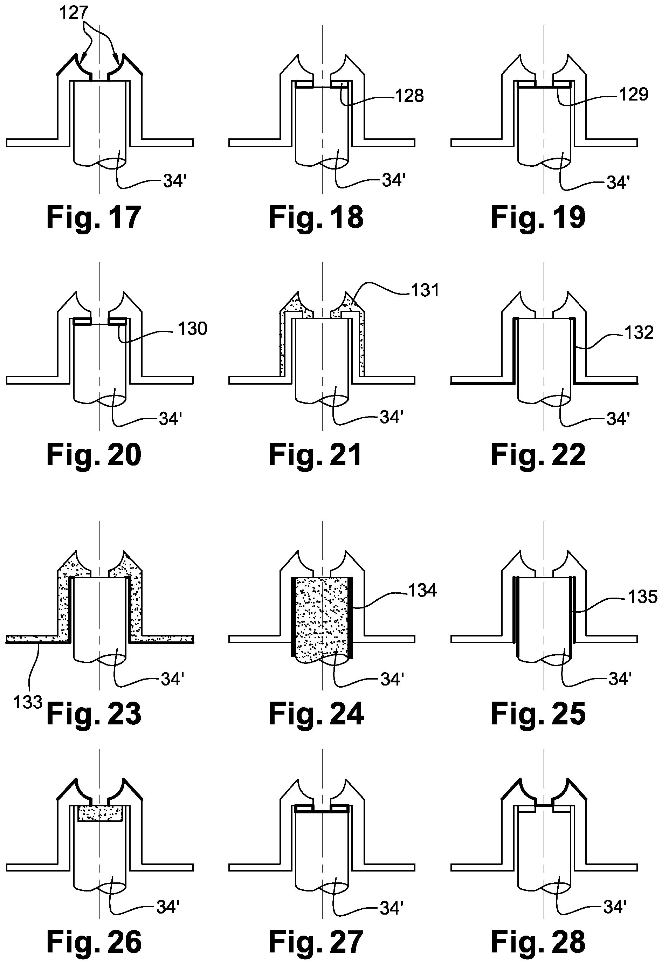

Sur la figure 17, la couche anti-bactérienne 127 s'étend uniquement sur le bord de la cavité sphérique et au-delà de l'orifice 58'.

Sur la figure 18, la couche anti-bactérienne 128 constitue une surépaisseur en saillie de la rondelle, orifice 58' exclu, en regard de la zone d'appui du pion 34' à l'exception du bord périphérique de ce dernier.

Sur la figure 19, la couche anti-bactérienne 129 a la même forme que précédemment mais est co-moulée avec la rondelle ou résulte d'un traitement anti-bactérien de la rondelle sur une partie de son épaisseur, exception faite de sa partie en regard du bord périphérique du pion 34'.

Sur la figure 20, la couche anti-bactérienne 130 a la même forme que précédemment mais est un insert revêtu extérieurement d'une couche de matière anti-bactérienne.

Sur la figure 21, la couche anti-bactérienne 131 est une double épaisseur massive de la membrane, lui donnant sa forme extérieure et recouvrant sa partie coiffant le pion 34'.

Sur la figure 22, la membrane est intégralement revêtue d'une couche d'une matière anti-bactérienne et cette couche est elle-même recouverte d'un isolant 132 en matériau non anti-bactérien dans ses parties situées en amont de la valve, bord extérieur du pion 34' exclu.

Sur la figure 23, la membrane est massivement constituée d'une matière anti-bactérienne et est recouverte d'un isolant en 133 matériau non anti-bactérien dans ses parties situées en amont de la valve, bord extérieur du pion 34' exclu.

Sur la figure 24, le pion 34' est constitué d'une matière anti-bactérienne et est recouvert d'un isolant 134 en matériau non anti-bactérien dans ses parties situées en amont de la valve.

Sur la figure 25, le pion 34' est recouvert d'une matière anti-bactérienne qui est elle-même recouverte d'un isolant 135 en matériau non anti-bactérien dans ses parties situées en amont de la valve.

Toutes les combinaisons possibles de ces variantes sont possibles, pour autant qu'elles entrent dans la définition de l'invention.

Par exemple, sur la figure 26, on a combiné les caractéristiques des variantes des figures 8 et 17. Sur la figure 27, on a combiné les caractéristiques des variantes des figures 5 et 18 et, sur la figure 28, on a combiné les caractéristiques des variantes des figures 6 et 17.

Il est bien entendu que les exemples de réalisation qui viennent d'être décrits ne présentent aucune limitation de la portée de l'invention et que d'autres variantes et combinaisons de variantes sont possibles.

## Revendications

1. Embout (10) de distribution de liquide destiné à équiper un contenant, ledit embout comprenant une valve procurant, au repos, une obturation du contenant en séparant un volume amont d'un volume aval, ladite valve étant capable de subir des manoeuvres d'ouverture par déformation élastique, ladite valve comprenant au moins deux éléments (34, 54 ; 34') mobiles l'un par rapport à l'autre, chaque élément comportant une zone d'appui (36, 55 ; 36') contre l'autre élément, agencée de manière que, la valve étant au repos, les zones d'appui (36, 55 ; 36') des éléments s'appuient au moins partiellement l'une contre l'autre en formant une barrière surfacique (100, 102, 104) séparant le volume amont du volume aval, l'un au moins des deux éléments (34, 54 ; 34') étant tel qu'après une ou plusieurs manoeuvres d'ouverture, ladite barrière surfacique (100, 102, 104) ne recouvre plus la même partie de la zone d'appui (36, 55 ; 36') de l'un au moins des éléments, au moins l'un des éléments portant une matière anti-microbienne (115 - 131) sur ou à proximité immédiate d'une partie de sa zone d'appui (36, 55 ; 36') qui est recouverte par la barrière surfacique, **caractérisé en ce que** toute surface de l'embout de distribution se trouvant en contact avec le volume amont lorsque la valve est au repos est exempte de matière anti-microbienne.

2. Embout selon la revendication 1, dans lequel la matière anti-bactérienne est présente sous la forme d'une couche (115, 116, 125, 126, 127) qui recouvre au moins partiellement la zone d'appui (36, 55 ; 36') d'un des éléments.

3. Embout selon la revendication 1, dans lequel la matière anti-bactérienne est présente sous la forme d'une surépaisseur (122, 123, 124) en saillie de la zone d'appui (36, 55 ; 36') d'un des éléments.

4. Embout selon la revendication 3, dans lequel la surépaisseur (122, 123, 124) occupe sensiblement l'épaisseur d'un orifice de la valve.

5. Embout selon l'une quelconque des revendications 1 à 4, dans lequel l'un des éléments a subi un traitement anti-bactérien sur une partie (118, 129) de son épaisseur.

6. Embout selon l'une quelconque des revendications 1 à 5, dans lequel l'un des éléments est co-moulé avec une matière anti-bactérienne (119, 123).

7. Embout selon l'une quelconque des revendications 1 à 6, dans lequel l'un des éléments porte un insert (120, 121, 122, 123, 124).

8. Embout selon la revendication 7, dans lequel l'insert (122, 123) est massif en matière anti-bactérienne.

9. Embout selon la revendication 7, dans lequel l'insert (121, 124) est revêtu extérieurement d'une couche de matière anti-bactérienne.

10. Embout selon la revendication 7, dans lequel l'insert est revêtu uniquement sur sa face tournée vers l'extérieur d'une couche de matière anti-bactérienne.

11. Embout selon l'une quelconque des revendications 7 à 10, dans lequel l'insert (120, 121) est affleurant avec la zone d'appui de l'élément.

12. Embout selon l'une quelconque des revendications 7 à 10, dans lequel l'insert (122, 123, 124) fait saillie de la zone d'appui de l'élément de manière à former un pion traversant un orifice (56') de la valve.

13. Embout selon l'une quelconque des revendications 1 à 12, dans lequel l'un des éléments (36', 54') est recouvert, sur une face portant de la matière anti-bactérienne, d'une couche (132, 133, 134) de matière isolante non anti-bactérienne dans ses parties situées en amont de la valve.

14. Embout selon l'une quelconque des revendications 1 à 13, dans lequel la distance entre la zone d'appui et la matière anti-microbienne est inférieure à 3 mm, et de préférence inférieure à 1 mm.

15. Récipient de distribution de liquide comportant un réservoir sur lequel est monté un embout de distribution (10) selon l'une quelconque des revendications 1 à 14.

## Patentansprüche

1. Ansatzstück (10) zur Abgabe einer Flüssigkeit für die Anbringung an einem Behälter, wobei das Ansatzstück ein Ventil umfasst, das, in Ruhestellung, einen Verschluss des Behälters bewirkt, indem es ein stromaufwärtiges Volumen von einem stromabwärtigen Volumen trennt, wobei das Ventil dazu eingerichtet ist, Betätigungen zum Öffnen durch elastische Verformung unterzogen zu werden, wobei das Ventil wenigstens zwei Elemente (34, 54; 34') umfasst, die relativ zueinander beweglich sind, wobei jedes Element eine Zone zur Abstützung (36, 55; 36') gegen das andere Element aufweist, die so ausgebildet ist, dass, wenn das Ventil in Ruhestellung ist, sich die Abstützzonen (36, 55; 36') wenigstens teilweise gegeneinander abstützen, indem sie eine oberflächliche Sperre (100, 102, 104) bilden, die das stromaufwärtige Volumen von dem stromabwärtigen Volumen trennt, wobei wenigstens eines der zwei Elemente (34, 54; 34') dergestalt ist, dass nach einer oder mehreren Öffnungsbetätigungen die oberflächliche Sperre (100, 102, 104) nicht mehr den selben Teil der Abstützzone (36, 55; 36') des wenigstens einen der Elemente bedeckt, wobei das wenigstens eine der Elemente auf oder in unmittelbarer Nähe eines Teils seiner Abstützzone (36, 55; 36'), das durch die oberflächliche Sperre (100, 102, 104) bedeckt ist, eine antimikrobielle Substanz (115-131) trägt, **dadurch gekennzeichnet, dass** die gesamte Oberfläche des Ausgabeansatzstücks, die mit dem stromaufwärtigen Volumen in Kontakt ist, wenn das Ventil in Ruhestellung ist, frei von antimikrobieller Substanz ist.

2. Ansatzstück nach Anspruch 1, wobei die antimikrobielle Substanz in Form einer Schicht (115, 116, 125, 126, 127) vorhanden ist, welche die Abstützzone (36, 55; 36') eines der Elemente wenigstens teilweise bedeckt.

3. Ansatzstück nach Anspruch 1, wobei die antimikrobielle Substanz in Form einer Überdicke (122, 123, 124) vorhanden ist, die von der Abstützzone (36, 55; 36') eines der Elemente vorspringt.

4. Ansatzstück nach Anspruch 3, wobei die Überdicke (122, 123, 124) im Wesentlichen die Dicke einer Öffnung des Ventils einnimmt.

5. Ansatzstück nach einem der Ansprüche 1 bis 4, wobei eines der Elemente auf einem Teil (118, 129) seiner Dicke einer antibakteriellen Behandlung unterzogen wurde.

6. Ansatzstück nach einem der Ansprüche 1 bis 5, wobei eines der Elemente mit einer antimikrobiellen Substanz (119, 123) zusammen geformt ist

7. Ansatzstück nach einem der Ansprüche 1 bis 6, wobei eines der Elemente einen Einsatz (120, 121, 122, 123, 124) trägt.

8. Ansatzstück nach Anspruch 7, wobei der Einsatz (122, 123) massiv aus antibakterieller Substanz ist.

9. Ansatzstück nach Anspruch 7, wobei der Einsatz (121, 124) außen mit einer Schicht aus antibakterieller Substanz beschichtet ist.

10. Ansatzstück nach Anspruch 7, wobei der Einsatz nur auf seiner nach außen gekehrten Seite mit einer Schicht aus antibakterieller Substanz beschichtet ist.

11. Ansatzstück nach einem der Ansprüche 7 bis 10, wobei der Einsatz (120, 121) mit der Abstützzone des Elements flächenbündig ist.

12. Ansatzstück nach einem der Ansprüche 7 bis 10, wobei der Einsatz (122, 123, 124) von der Abstützzone des Elements so vorspringt, dass er einen Stift bildet, der eine Öffnung (56') des Ventils durchquert.

13. Ansatzstück nach einem der Ansprüche 1 bis 12, wobei eines der Elemente (36', 54') auf einer Seite, welche die antibakterielle Substanz trägt, in seinen Teilen, die sich stromaufwärts zu dem Ventil befinden, mit einer isolierenden Schicht (132, 133, 134) aus nichtantibakterieller Substanz bedeckt ist.

14. Ansatzstück nach einem der Ansprüche 1 bis 13, wobei die Distanz zwischen der Abstützzone und der antibakteriellen Substanz kleiner als 3 mm und vorzugsweise kleiner als 1 mm ist.

15. Behälter zur Abgabe von Flüssigkeit mit einem Tank, an dem ein Abgabeansatzstück (10) nach einem der Ansprüche 1 bis 14 montiert ist.

## Claims

1. Tip (10) for dispensing liquid designed to be fitted on a container, said tip comprising a valve which seals the container by separating an upstream volume from a downstream volume in the non-operating position, said valve being able to withstand opening manoeuvres by elastic deformation, said valve comprising at least two elements (34, 53; 34') that are mobile relative to one another, each element having a bearing zone (36, 55; 36') against the other element, disposed so that when the valve is in the non-operating position, the bearing zones (36, 55; 36') of the elements bear at least partially one another forming a surface barrier (100, 102, 104) separating the upstream volume from the downstream volume, at least one of the two elements (34, 54; 34') being such that after one or more opening manoeuvres said surface barrier (100, 102, 104) no longer covers the same part of the bearing zone (36, 55; 36') of at least one of the elements, at least one of the elements bearing an anti-microbial material (115 - 131) on or in the immediate vicinity of a part of its bearing zone (36, 55; 36') which is covered by the surface barrier, **characterised in that** the entire surface of the dispensing tip in contact with the upstream volume when the valve is in the non-operating position is free of anti-microbial material.

2. Tip according to claim 1, wherein the anti-bacterial material is present in the form of a layer (115, 116, 125, 126, 127) which at least partially covers the bearing zone (36, 55; 36') of one of the elements.

3. Tip according to claim 1, wherein the anti-bacterial material is present in the form of an excess thickness (122, 123, 124) protruding from the bearing zone (35, 55; 36') of one of the elements.

4. Tip according to claim 3, wherein the excess thickness (122, 123, 124) substantially occupies the thickness of an orifice of the valve.

5. Tip according to any one of claims 1 to 4, wherein one of the elements has undergone an anti-bacterial treatment on a part (118, 129) of its thickness.

6. Tip according to any one of claims 1 to 5, wherein one of the elements is co-moulded with an anti-bacterial material (119, 123).

7. Tip according to any one of claims 1 to 6, wherein one of the elements bears an insert (120, 121, 122, 123, 124).

8. Tip according to claim 7, wherein the insert (122, 123) is made of bulk anti-bacterial material.

9. Tip according to claim 7, wherein the insert (121, 124) is externally coated with a layer of anti-bacterial material.

10. Tip according to claim 7, wherein the insert is coated with a layer of anti-bacterial material solely on its face directed towards the exterior.

11. Tip according to any one of claims 7 to 10, wherein the insert (120, 121) is flush with the bearing zone of the element.

12. Tip according to any one of claims 7 to 10, wherein the insert (122, 123, 124) protrudes from the bearing zone of the element so as to form a pin passing through an orifice (56') of the valve.

13. Tip according to any one of claims 1 to 12, wherein one of the elements (36', 54') is coated, on a face bearing the anti-bacterial material, with a layer (132, 133, 134) of non anti-bacterial insulating material in its parts located upstream of the valve.

14. Tip according to any one of claims 1 to 13, wherein the distance between the bearing zone and the anti-microbial material is less than 3 mm and preferably less than 1 mm.

15. Recipient for dispensing liquid including a reservoir on which a dispensing tip (10) according to any one of claims 1 to 14 is mounted.
